(19) Europäisches Patentamt
European Patent Office
Office européen des brevets

(11) Veröffentlichungsnummer : **0 167 945**
**B1**

(12) # EUROPÄISCHE PATENTSCHRIFT

(45) Veröffentlichungstag der Patentschrift : **13.04.88**

(51) Int. Cl.⁴ : **C 07 C 97/10**, A 61 K 31/135

(21) Anmeldenummer : **85108063.0**

(22) Anmeldetag : **28.06.85**

(54) **Phenylethylaminopropiophenon-Derivate, Verfahren zu ihrer Herstellung und diese enthaltende Arzneimittel.**

(30) Priorität : **11.07.84 DE 3425452**

(43) Veröffentlichungstag der Anmeldung : **15.01.86 Patentblatt 86/03**

(45) Bekanntmachung des Hinweises auf die Patenterteilung : **13.04.88 Patentblatt 88/15**

(84) Benannte Vertragsstaaten : **AT BE CH DE FR GB IT LI NL SE**

(56) Entgegenhaltungen :
**DE-A- 2 110 545**
**DE-A- 2 160 911**
**DE-B- 1 493 574**

(73) Patentinhaber : **Troponwerke GmbH & Co. KG**
**Berliner Strasse 156**
**D-5000 Köln 80 (DE)**

(72) Erfinder : **Seidel, Peter-Rudolf, Dr.**
**Alte Heide 5d**
**D-5000 Köln 90 (DE)**
Erfinder : **Junge, Bodo, Dr.**
**Spiekern 23**
**D-5600 Wuppertal 23 (DE)**
Erfinder : **Horstmann, Harald, Dr.**
**Claudiusweg 19**
**D-5600 Wuppertal 1 (DE)**
Erfinder : **Traber, Jörg, Dr.**
**Löwenburgstrasse 12**
**D-5204 Lohmar 21 (DE)**
Erfinder : **Davies, Margaret A., Dr.**
**Thebäerstrasse 80**
**D-5000 Köln 30 (DE)**
Erfinder : **Schuurman, Teunis, Dr.**
**Rohrbergstrasse 20**
**D-5204 Lohmar 21 (DE)**

(74) Vertreter : **Adrian, Albert, Dr. et al**
**c/o BAYER AG Konzernverwaltung RP Patentabteilung**
**D-5090 Leverkusen 1, Bayerwerk (DE)**

EP 0 167 945 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue Phenylethylaminopropiophenon-Derivate und deren Salze mit physiologisch unbedenklichen Säuren, Verfahren zu hirer Herstellung sowie ihre Verwendung bei der Bekämpfung von Krankheiten, insbesondere ihre Verwendung zur Behandlung von Erkrankungen des zentralen Nervensystems.

Die Erfindung betrifft Phenylethylaminopropiophenon-Derivate der allgemeinen Formel (I)

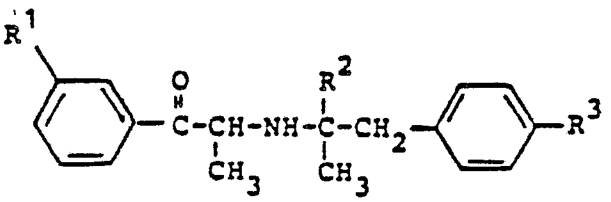

(I)

in welcher

$R^1$ für Wasserstoff, gegebenenfalls durch Fluor substituiertes niederes Alkyl oder Halogen steht,

$R^2$ Wasserstoff oder niederes Alkyl und

$R^3$ Wasserstoff, Alkoxy oder Halogen bedeuten, sowie ihre physiologisch verträglichen Salze mit anorganischen oder organischen Säuren.

Es ist bekannt, daß es für Moleküle mit einem Asymmetriezentrum zwei Enantiomere und für Moleküle mit zwei Asymmetriezentren zwei Diastereoisomere und damit zwei Enantiomerenpaare gibt.

Die erfindungsgemäßen Phenylethylaminopropiophenon-Derivate der allgemeinen Formel (I) besitzen zwei Asymmetriezentren bzw. ein Asymmetriezentrum, demgemäß sollen alle optischen Isomere und Diastereoisomere zum Gegenstand der vorliegenden Erfindung gehören.

Die neuen erfindungsgemäßen Verbindungen der allgemeinen Formel (I) zeigen eine ausgeprägte Wirkung auf das zentrale Nervensystem.

Die erfindungsgemäßen substituierten Phenylethylaminopropiophenone sind durch die Formel (I) allgemein definiert. Bevorzugt sind diejenigen Verbindungen der Formel (I), in denen

$R^1$ für Wasserstoff, niederes Alkyl mit 1 bis 2 Kohlenstoffatomen, gegebenenfalls substituiert durch Fluor, oder Halogen, vorzugsweise Fluor oder Chlor, steht,

$R^2$ für Wasserstoff oder niederes Alkyl mit 1 bis 2 Kohlenstoffatomen steht, und

$R^3$ für Wasserstoff, Alkoxy mit 1 bis 2 Kohlenstoffatomen oder Halogen, vorzugsweise Chlor oder Fluor, steht.

Die vorliegende Erfindung betrifft weiterhin ein Verfahren zur Herstellung von Verbindungen der allgemeinen Formel (I), dadurch gekennzeichnet, daß man Halogenketone der allgemeinen Formel (II),

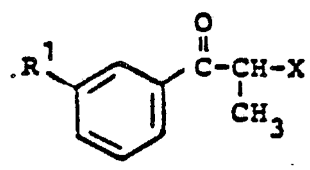

(II)

in welcher

$R^1$ die oben angegebene Bedeutung hat und

X Halogen, vorzugsweise Brom, bedeutet

mit Aminen der allgemeinen Formel (III)

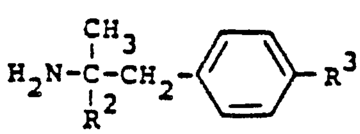

(III)

in welcher $R^2$ und $R^3$ die oben angegebene Bedeutung haben, in Gegenwart von inerten Lösungsmitteln bei Temperaturen zwischen 0° und 150 °C, gegebenenfalls in Gegenwart eines Protonenacceptors, umsetzt und die so erhaltenen Verbindungen der Formel (I) gegebenenfalls anschließend in bekannter Weise in die Säureadditionssalze überführt.

Die erfindungsgemäßen Wirkstoffe sind neu und zeigen im Tierversuch eine ausgeprägte Wirkung auf das zentrale Nervensystem. Sie sind wirksam in Versuchsanordnungen, mit denen anxiolytische, nootrope, insbesondere aber antidepressive Wirkungen gefunden werden. Daher eignen sie sich insbesondere zur Behandlung von depressiven Zuständen und stellen somit eine Bereicherung des Arzneimittelschatzes dar.

Die neuen Wirkstoffe besitzen außerdem analgetische und antiphlogistische Wirkungen, die z. B. am Carrageenin-Pfotenödem bei Ratten nachgewiesen werden konnten.

Bevorzugt sind Verbindungen der Formel I, in denen

$R^1$ für Wasserstoff, Halogen, besonders vorzugsweise Fluor oder Chlor, oder Trifluormethyl steht,

$R^2$ für Wasserstoff oder Methyl und

$R^3$ für Wasserstoff, Methoxy oder Halogen, besonders vorzugsweise für Chlor steht.

Die Herstellung von Verbindungen der allgemeinen Formel (I) wird beispielsweise durch das folgende Formelschema erläutert :

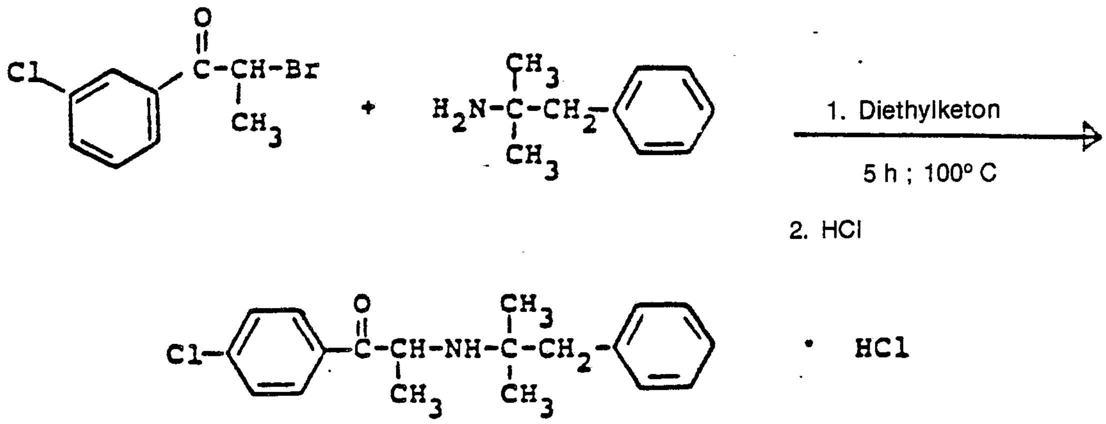


Die Alkylierungsreaktion erfolgt in Lösungsmitteln, die gegenüber den Reaktionspartnern inert sind. Hierzu gehören vorzugsweise Ether, wie Diethylether, Diisopropylether, Tetrahydrofuran, Dioxan ; Kohlenwasserstoffe, wie Ligroin, Hexan, Cyclohexan, Benzol, Toluol, Xylol, Tetralin ; Halogenkohlenwasserstoffe, wie Chloroform, Dichlormethan, Tetrachlorkohlenstoff, 1,2-Dichlorethan, Chlorbenzol ; Ketone, wie Aceton, Methylethylketon, Diethylketon, Methylbutylketone ; Nitrile, wie Acetonitril, Propionitril ; Carbonsäureamide, wie Demithylformamid, N-Methylpyrrolidon, Dimethylsulfoxid ; ferner handelsübliche technische Gemische dieser Lösungsmittel, besonders vorzugsweise Diethylketon.

Die Umsetzung kann in einem Überschuß der eingesetzten Verbindungen der allgemeinen Formeln (II) und/oder (III) und gegebenenfalls in Gegenwart eines säurebindenden Mittels, z. B. eines Alkoholates, wie Kalium-tert.-butylat oder Natriummethylat, eines Alkalihydroxids, wie Natrium- oder Kaliumhydroxid, eines Erdalkalihydroxids, wie Calcium- oder Bariumhydroxid, eines Alkali- oder Erdalkalicarbonates, wie Natriumcarbonat, Kaliumcarbonat, Calciumcarbonate, Natrium- oder Kaliumhydrogencarbonat oder tertiärer organischer Basen, wie Triethylamin, Ethyldiisopropylamin, N,N-Dimethylanilin, Pyridin, Chinolin oder Isochinolin, zweckmäßigerweise bei Temperaturen zwischen 0° und 150 °C, vorzugsweise bei Temperaturen zwischen 20° und 120 °C, z. B. bei der Siedetemperatur des verwendeten Lösungsmittels, durchgeführt werden. Die Umsetzung kann jedoch auch ohne Lösungsmittel durchgeführt werden. Besonders vorteilhaft wird die Umsetzung jedoch in Diethylketon in Gegenwart von Kaliumcarbonat durchgeführt.

Die Umsetzung wird normalerweise bei Atmosphärendruck durchgeführt.

Bei der Durchführung des erfindungsgemäßen Verfahrens setzt man auf 1 Mol der Verbindung der Formel (II) mindestens 1 Mol des Amins der Formel (III) und mindestens 1 Mol eines der genannten säurebindenden Mittel ein. Vorzugsweise verwendet man auf 1 Mol der Verbindung der Formel (II) 2 bis 3 Mol des Amins der Formel (III).

Zweckmäßigerweise kann die Umsetzung auch unter Inertgas, wie z. B. Stickstoff oder Argon, durchgeführt werden.

Die Aufarbeitung erfolgt zweckmäßig durch Eindampfen der Reaktionslösung, Aufnehmen des Konzentrats in einem geeigneten Lösungsmittel, wie Diethylether oder Methylenchlorid, und Reinigen, gegebenenfalls mit Hilfe der Chromatographie an Kieselgel bzw. Aluminiumoxid oder anderen geeigneten Absorbentien.

Die als Vorprodukte einzusetzenden Halogenketone der allgemeinen Formel (II) sind bekannt und können nach bekannten Methoden hergestellt werden.

Es wurden folgende Bromketone der allgemeinen Formel (III) verwendet :

α-Brompropiophenon (P.S. Bailey et al., J. Amer. Chem. Soc. 71, 2886 (1949)) ;

3-Chlor-α-brompropiophenon (L. Szotyory et al., J. Prakt. Chem. 23, 202 (1963) ; C.A. 60, 4045 (1964)) ;

3-Fluor-α-brompropiophenon (US-Patent 3 885 046) ;

3-Trifluormethyl-α-brompropiophenon (I.J. Borowitz et al., J. Amer. Chem. Soc. 94, 6817 (1972)) :

Die als Vorprodukte einzusetzenden Amine der allgemeinen Formel (III) sind bekannt und teilweise im

Handel erhältlich. Ihre Herstellung wird in zahlreichen Literaturbeispielen beschrieben (vgl. zum Beispiel W.H. Hartung et al., J. Amer. Chem. Soc. 53, 1875 (1931) ; G.B. Bachman et al., J. Amer. Chem. Soc. 76, 3972 (1954) ; US-Patente 2 408 345 und 2 590 079).

Von ganz besonderer Bedeutung sind Verbindungen der allgemeinen Formel (I), in welcher
$R^1$ für Wasserstoff, Trifluormethyl, Fluor oder Chlor steht,
$R^2$ für Methyl und
$R^3$ für Wasserstoff, Methoxy oder Chlor steht.

Als neue erfindungsgemäße Wirkstoffe seien im einzelnen genannt :

1-(3-Chlorphenyl)-2-(1-methyl-2-phenylethylamino)propan-1-onhydrochlorid ;
1-Phenyl-2-(1,1-dimethyl-2-phenylethylamino)propan-1-onhydrochlorid ;
1-(3-Trifluormethylphenyl)-2-(1,1-dimethyl-2-phenylethylamino)propan-1-onhydrochlorid ;
1-(3-Fluorphenyl)-2-(1,1-dimethyl-2-phenyl-ethylamino)-propan-1-onhydrochlorid ;
1-(3-Chlorphenyl)-2-(1,1-dimethyl-2-phenylethylamino)-propan-1-onhydrochlorid ;
1-(3-Chlorphenyl)-2-(1,1-dimethyl-2-(4-chlorphenyl)-ethylamino)propan-1-onhydrochlorid ;
1-(3-Chlorphenyl)-2-(1,1-dimethyl-2-(4-methoxyphenyl)-ethylamino)propan-1-onhydrochlorid.

Die Verbindungen wurden auf anxiolytische, nootrope und antidepressive Eigenschaften geprüft. Als Leittest für die anxiolytische Wirkung diente die Antagonisierung der Fußschock-induzierten Aggressivität nach Tedeschi[1] ; für die antidepressive Wirkung der Antitetrabenazin[2]-und Amphetaminpotenzierungstest[2]. Beispielsweise wurden für die antidepressiven Wirkungen der erfindungsgemäßen Substanzen folgende Werte erhalten :

1) Tetrabenazin Antagonismus

Antidepressiv wirksame Substanzen antagonisieren die durch Tetrabenazin induzierte Ptosis bei Maüsen. Der $DE_{50}$-Wert gibt diejenige Dosis an, bei der die durch 20 mg/kg i.p. Tetrabenazin induzierte Ptosis zu 50 % reduziert ist. Dazu seien Beispiele genannt :

| Verbindung | $DE_{50}$ (mg/kg i.p.) |
|---|---|
| Beispiel 1 | 1,0 |
| Beispiel 2 | 14,0 |
| Beispiel 3 | 12,0 |
| Beispiel 4 | 19,0 |
| Beispiel 5 | 6,5 |
| Beispiel 6 | 6,0 |
| Beispiel 7 | 10,0 |

2) Amphetamin-Potenzierung

Antidepressiv wirksame Substanzen potenzieren das Amphetamininduzierte stereotype Verhalten bei der Ratte.

Der angegebene $DE_{50}$-Wert ist die Dosis, bei der das Amphetamininduzierte Verhalten nach Gabe von 2 mg/kl DL-Amphetaminsulfat i. v. um 50 % verstärkt ist. Dazu seien Beispiele genannt :

| Verbindung | $DE_{50}$ (mg/kg i.p.) |
|---|---|
| Beispiel 1 | 13,0 |
| Beispiel 2 | 6,0 |
| Beispiel 3 | 9,0 |

Zur vorliegenden Erfindung gehören pharmazeutische Zubereitungen, die neben nicht-toxischen, inerten, pharmazeutisch geeigneten Trägerstoffen eine oder mehrere der erfindungsgemäßen Verbindungen oder deren Salze enthalten oder die aus einer oder mehrerer der erfindungsgemäßen Verbindungen oder deren Salzen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z. B. Tabletten, Dragees, Kapseln, Pillen,

1 Tedeschi et al., J. Pharmacol. Exp. Ther. 129, 28-34, 1959

2 J. L. Howard et al., in : Antidepressants : Neurochemical, Behavioral and Clinical Perspectives, herausgegeben von S. J. Enna et al., Raven Press N. Y., S. 107-120, 1981.

Suppositorien und Ampullen vorliegen, deren Wirkstoffgehalt einem Bruchteil oder einem Vielfachen einer Einzeldosis entspricht. Die Dosierungseinheiten können z. B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten, pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen genannt.

Tabletten, Dragees, Kapseln, Pillen und Granulate können den oder die Wirkstoffe neben den üblichen Trägerstoffen enthalten, wie (a) Füll- und Streckmittel, z. B. Stärken, Milchzucker, Rohrzucker, Glukose, Mannit und Kieselsäure, (b) Bindemittel, z. B. Carboxymethylcellulose, Aliginate, Gelatine, Polyvinylpyrrolidon, (c) Feuchthaltemittel, z. B. Glycerin, (d) Sprengmittel, z. B. Agar-Agar, Calciumcarbonat und Natriumbicarbonat, (e) Lösungsverzögere, z. B. Paraffin und (f) Resorptionsbeschleuniger, z. B. quarternäre Ammoniumverbindungen, (g) Netzmittel, z. B. Cetylalkohol, Glycerinmonostearat, (h) Adsorptionsmittel, z. B. Kaolin und Bentonit und (i) Gleitmittel, z. B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opalisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z. B. Polyethylenglykole, Fette, z. B. Kakaofett, und höhere Ester (z. B. $C_{14}$-Alkohol mit $C_{16}$-Fettsäure) oder Gemische dieser Stoffe.

Lösungen und Emulsionen können neben dem oder den Wirkstoffen die üblichen Trägerstoffe wie Lösungsmittel, Lösungsvermittler und Emulgator, z. B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinfomal, Tetrahydrofurfurylalkohol, Poyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z. B. Wasser, Ethylalkohol, Propylenglykol, Suspendiermittel, z. B. ethoxylierte Isostearylalkohole, Polyoxyethylensorbit- und -sorbitanester, mikrokristalline Cellulose, Aluminiummetahydroxid, Bentonid, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z. B. Pfefferminzöl und Eukalyptusöl und Süßmittel, z. B. Saccharin, enthalten.

Die terapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung, vorhanden sein.

Die oben aufgeführten pharmazeutischen Zubereitungen können außer Verbindungen der Formel (I) und/oder deren Salzen auch andere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Zur vorliegenden Erfindung gehören auch die Verwendung der Verbindungen for Formel (I) und/oder deren Salzen sowie von pharmazeutischen Zubereitungen, die eine oder mehrere Verbindungen der Formel (I) und/oder deren Salze enthalten, in der Humanmedizin zur Verhütung, Besserung und/oder Heilung der oben aufgeführten Erkrankungen.

Die Wirkstoffe oder die pharmazeutischen Zubereitungen können vorzugsweise oral, parenteral und/oder rectal, vorzugsweise oral und parenteral, insbesondere oral und intravenös, appliziert werden.

Im allgemeinen hat es sich als vorteilhaft erwiesen, den oder die Wirkstoffe bei parenteraler (i. v. oder i. m.) Applikation in Mengen von etwa 0,01 bis etwa 10, vorzugsweise von 0,1 bis 1 mg/kg Körpergewicht je 24 Stunden und bei oraler Applikation in Mengen von etwa 0,05 bis etwa 100, vorzugsweise 0,1 bis 10 mg/kg Körpergewicht je 24 Stunden, gegebenenfalls in Form mehrerer Einzelgaben zur Erzielung der gewünschten Ergebnisse zu verabreichen. Eine Einzelgabe enthält den oder die Wirkstoffe, vorzugsweise in Mengen von etwa 0,01 bis etwa 30, insbesondere 0,03 bis 3 mg/kg Körpergewicht.

Es kann jedoch erforderlich sein, von den genannten Dosierungen abzuweichen, und zwar in Abhängigkeit von der Art und dem Köpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit

weniger als der o. g. Menge Wirkstoff auszukommen, während in anderen Fällen die oben angeführte Wirkstoffmenge überschritten werden muß. Die Festlegung der jeweils erforderlichen optimalden Dosierung und Applikationsart der Wirkstoffe kann durch jeden Fachmann aufgrund seines Fachwissens leicht erfolgen.

Die vorliegende Erfindung soll durch die nachfolgenden Beispiele noch näher erläutert werden :

Beispiel 1

Darstellung von 1-(3-Chlorphenyl)-2-(1,1-dimethyl-2-phenylethylamino)propan-1-onhydrochlorid

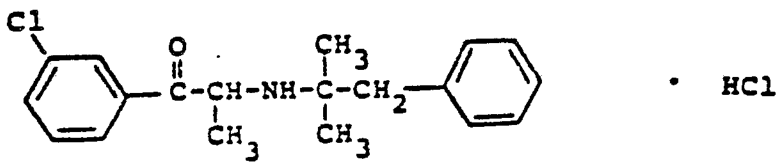

0,05 Mol 3-Chlor-α-brompropiophenon werden zusammen mit 0,15 Mol 1-Phenyl-2-methyl-2-aminopropan in 100 ml Diethylketon unter $N_2$-Atmospäre 5 Stunden auf 100 °C erhitzt. Anschließend wird das Lösungsmittel abgedampft, der Rückstand in 2n HCl aufgenommen und mit Diethylether ausgeschüttelt. Die wäßrige salzsaure Phase wird mit festem $NaHCO_3$ versetzt, wonach die sich ausscheidende Base in Diethylether aufgenommen und als Hydrochlorid durch Zugabe einer Diethylether-HCl-Lösung erhalten wird.

Ausbeute : 83 % der Theorie ; Fp 193 °C (Hydrochlorid).

(Siehe Tabelle Seite 7 f.)

Analog wurden dargestellt :

| Beispiel Nr. | Formel | Fp (0°C) | Ausbeute (% d. Th.) |
|---|---|---|---|
| 2 | 1-(3-Chlorphenyl)-2-(1-methyl-2-phenylethylamino) propan-1-onhydrochlorid 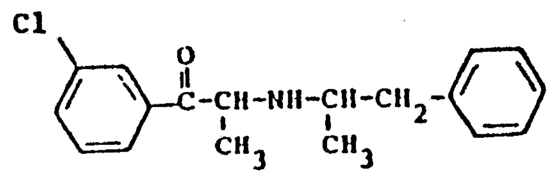 · HCl | 187 | 12 |
| 3 | 1-(3-Chlorphenyl)-2-(1,1-dimethyl-2-(4-chlorphenyl)ethylamino)propan-1-onhydrochlorid 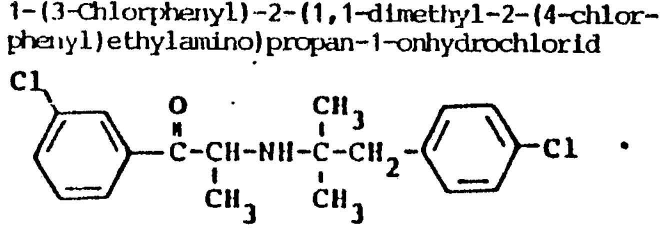 · HCl | 189 | 70 |
| 4 | 1-Phenyl-2-(1,1-dimethyl-2-phenylethylamino)-propan-1-onhydrochlorid 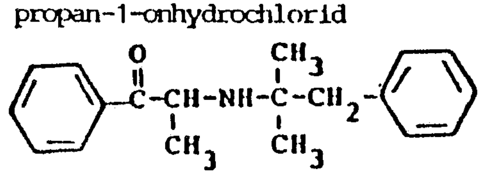 | 128 | 69. |

Beispiel 5

Darstellung von 1-(3-Chlorphenyl)-2-(1,1-dimethyl-2-(4-methoxyphenyl)ethylamino)propan-1-onhydrochlorid

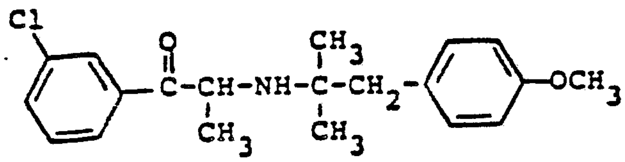 • HCl

Zu einer Lösung von 0,02 Mol 3-Chlor-α-brompropiophenon und 0,02 Mol 1-(4-Methoxyphenyl)-2-methyl-2-aminopropan in 30 ml Diethylketon gibt man 0,02 Mol $K_2CO_3$ und kocht 4 Stunden unter Rückfluß. Anschließend wird das Lösungsmittel abgedampft, der Rückstand in Methylenchlorid aufgenommen und mit Wasser gewaschen. Man reinigt die in der Methylenchloridphase enthaltene Base durch Säulenchromatografie an Kieselgel 60 (Merck). Das durch Eluieren mit einem Essigsäureethylester-Methylenchlorid-Gemisch (2 : 8) erhaltene Amin wird aus Diethylether durch Einleiten von HCl-Gas in das Hydrochlorid umgewandelt.

Ausbeute : 50 % der Theorie ; Fp. 103 °C (Hydrochlorid).

Beispiel 6

Darstellung von 1-(3-Fluorphenyl)-2-(1,1-dimethyl-2-phenylethylamino)propan-1-onhydrochlorid

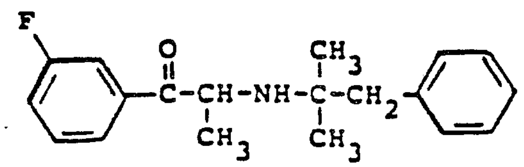 • HCl

0,05 Mol 3-Fluor-α-brompropiophenon und 0,15 Mol 1-Phenyl-2-methyl-2-aminopropan werden in 100 ml Diethylketon in Gegenwart von 0,1 Mol fein gepulvertem $K_2CO_3$ 24 Stunden bei 20-25 °C gerührt.

Die Aufarbeitung erfolgt wie im Beispiel 1 beschrieben.

Ausbeute : 61 % der Theorie ; Fp 181 °C (Hydrochlorid).

Beispiel 7

Darstellung von 1-(3-Trifluormethylphenyl)-2-(1,1-dimethyl-2-phenylethylamino)propan-1-onhydrochlorid

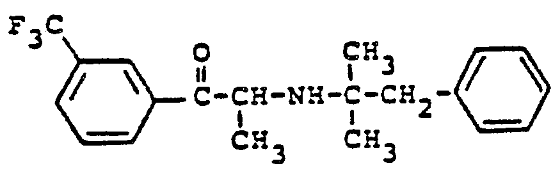 • HCl

0,05 Mol 3-(Trifluormethylphenyl)-α-brompropiophenon und 0,15 Mol 1-Phenyl-2-methyl-2-aminopropan werden in 100 ml Diethylketon 72 Stunden bei 25 °C gerührt. Danach wird das Lösungsmittel abdestilliert, der Rückstand in Methylenchlorid aufgenommen und an Kieselgel 60 (Merck) chromatografisch gereinigt, wobei als Elutionsmittel ein Methylenchlorid-Methanol-Gemisch (98 : 2) verwendet wird. Die reine, ölig anfallende Base wird mit einer Diethylether-HCl-Lösung versetzt, im Vakuum eingeengt und durch Zugabe von wenig Diethylether das Hydrochlorid zur Kristallisation gebracht.

Ausbeute : 37 % der Theorie ; Fp 177 °C (Hydrochlorid).

**Patentansprüche**

1. Phenylethylaminopropiophenon-Derivate der allgemeinen Formel (I)

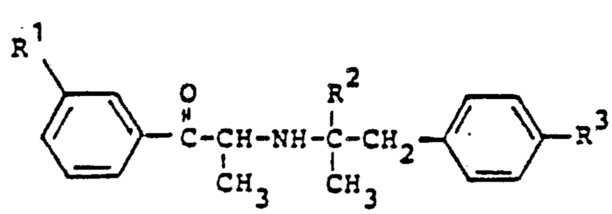 (I)

in welcher

$R^1$ für Wasserstoff, gegebenenfalls durch Fluor substituiertes niederes Alkyl oder Halogen steht,
$R^2$ Wasserstoff oder niederes Alkyl und
$R^3$ Wassserstoff, Alkoxy oder Halogen bedeuten,

sowie ihre physiologisch verträglichen Salze mit anorganischen oder organischen Säuren.

2. Phenylethylaminopropiophenon-Derivate nach Anspruch 1, in denen

$R_1$ für Wasserstoff, niederes Alkyl mit 1 bis 2 Kohlenstoffatomen, gegebenenfalls substituiert durch Fluor, oder Fluor oder Chlor steht,
$R^2$ für Wasserstoff oder niederes Alkyl mit 1 bis 2 Kohlenstoffatomen steht, und
$R^3$ für Wasserstoff, Alkoxy mit 1 bis 2 Kohlenstoffatomen oder Chlor oder Fluor steht.

3. Phenylethylaminopropiophenon-Derivate nach Anspruch 1, in denen

$R^1$ für Wasserstoff, Fluor, Chlor oder Trifluormethyl steht,
$R^2$ für Wasserstoff oder Methyl und
$R^3$ für Wasserstoff, Methoxy oder Chlor steht.

4. Phenylethylaminopropiophenon-Derivate nach Anspruch 1 aus der Gruppe bestehend aus

1-(3-Chlorphenyl)-2-(1-methyl-2-phenylethylamino)-propan-1-onhydrochlorid ;
1-Phenyl-2-(1,1-dimethyl-2-phenylethylamino)propan-1-onhydrochlorid ;
1-(3-Trifluormethylphenyl)-2-(1,1-dimethyl-2-phenylethylamino)propan-1-onhydrochlorid ;
1-(3-Fluorphenyl)-2-(1,1-dimethyl-2-phenyl-ethylamino)-propan-1-onhydrochlorid ;
1-(3-Chlorphenyl)-2-(1,1-dimethyl-2-phenylethylamino)-propan-1-onhydrochlorid ;
1-(3-Chlorphenyl)-2-(1,1-dimethyl-2-(4-chlorphenyl)-ethylamino)propan-1-onhydrochlorid und
1-(3-Chlorphenyl)-2-(1,1-dimethyl-2-(4-methoxyphenyl)-ethylamino)propan-1-onhydrochlorid.

5. Verfahren zur Herstellung von Phenylethylaminopropiophenon-Derivaten der allgemeinen Formel (I)

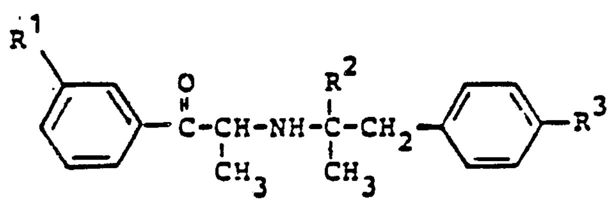

(I)

dadurch gekennzeichnet, daß man Halogenketone der allgemeinen Formel (II)

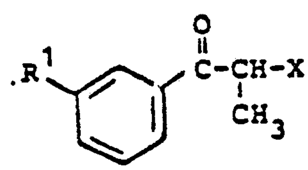

(II)

in welcher

$R^1$ die oben angegebene Bedeutung hat und
X Halogen, vorzugsweise Brom, bedeutet

mit Aminen der allgemeinen Formel (III)

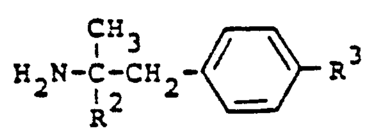

(III)

in welcher $R^2$ und $R^3$ die oben angegebene Bedeutung haben, in Gegenwart von inerten Lösungsmitteln bei Temperaturen zwischen 0° und 150 °C, gegebenenfalls in Gegenwart eines Protonenacceptors, umsetzt und die so erhaltenen Verbindungen der Formel (I) gegebenenfalls anschließend in bekannter Weise in die Säureadditionssalze überführt.

6. Verfahren nach Anspruch 5, dadurch gekennzeichnet, daß man als insertes Lösungsmittel Ether, Kohlenwasserstoffe, Halogenkohlenwasserstoffe, Ketone, Nitrile, Carbonamide oder handelsübliche technische Gemische dieses Lösungsmittel einsetzt.

7. Verfahren nach Anspruch 5, dadurch gekennzeichnet, daß man auf 1 Mol der Verbindung (II) mindestens 1 Mol des Amins der Formel (III) und mindestens 1 Mol eines säurebindenden Mittels einsetzt.

8. Arzneimittel enthaltend mindestens ein Phenylethylaminopropiopenon-Derivat der Formel (I)

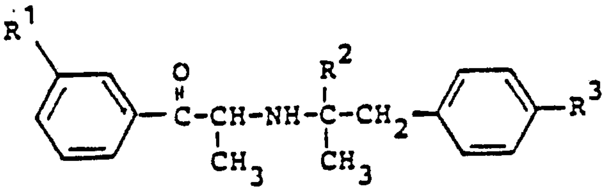

(I)

9. Verwendung von Phenylethylaminopropiophenon-Derivaten der Formel (I)

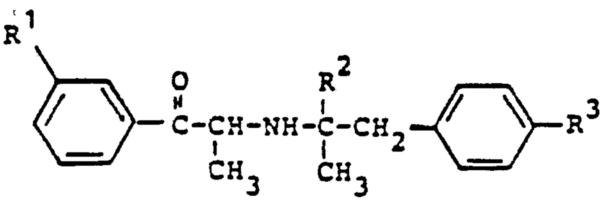

(I)

zur Herstellung von Arzneimitteln.

10. Phenylethylaminopropiophenon-Derivaten der Formel (I)

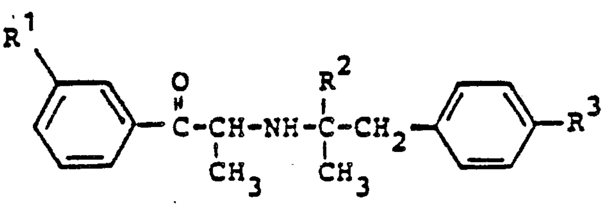

(I)

zur Verwendung bei der Bekämpfung von Erkrankungen und Störungen des zentralen Nervensystems.

**Claims**

1. Phenylethylaminopropiophenone derivatives of the general formula (I)

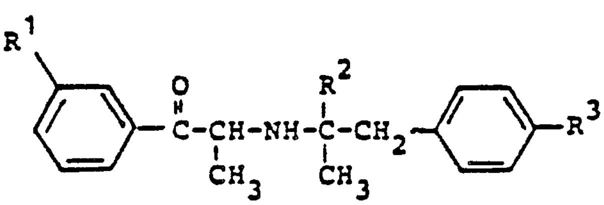

(I)

in which

$R^1$ represents hydrogen, lower alkyl which is optionally substituted by fluorine, or halogen,

$R^2$ denotes hydrogen or lower alkyl and

$R^3$ denotes hydrogen, alkoxy or halogen,

and their physiologically acceptable salts with inorganic or organic acids.

2. Phenylethylaminopropiophenone derivatives according to Claim 1, in which

$R^1$ represents hydrogen, lower alkyl with 1 or 2 carbon atoms which is optionally substituted by fluorine, or fluorine or chlorine,

$R^2$ represents hydrogen or lower alkyl with 1 or 2 carbon atoms, and

$R^3$ represents hydrogen, alkoxy with 1 or 2 carbon atoms or chlorine or fluorine.

3. Phenylethylaminopropiophenone derivatives according to Claim 1, in which

$R^1$ represents hydrogen, fluorine, chlorine or trifluoromethyl,

$R^2$ represents hydrogen or methyl and

$R^3$ represents hydrogen, methoxy or chlorine.

4. Phenylethylaminopropiophenone derivatives according to Claim 1, from the group comprising

1-(3-chlorophenyl)-2-(1-methyl-2-phenylethylamino)-propan-1-one hydrochloride ;

1-phenyl-2-(1,1-dimethyl-2-phenylethylamino)propan-1-one hydrochloride ;

1-(3-trifluoromethyl)-phenyl)-2-(1,1-dimethyl-2-phenylethylamino)propan-1-one hydrochloride ;

1-(3-fluorophenyl)-2-(1,1-dimethyl-2-phenyl-ethylamino)-propan-1-one hydrochloride ;

1-(3-chlorophenyl)-2-(1,1-dimethyl-2-phenylethylamino)-propan-1-one hydrochloride ;

1-(3-chlorophenyl)-2-(1,1-dimethyl-2-(4-chlorophenyl)-ethylamino)propan-1-one hydrochloride and

1-(3-chlorophenyl)-2-(1,1-dimethyl-2-(4-methoxyphenyl)-ethylamino)propan-1-one hydrochloride.

5. Process for the preparation of phenylethylaminopropiophenone derivatives of the general formula (I)

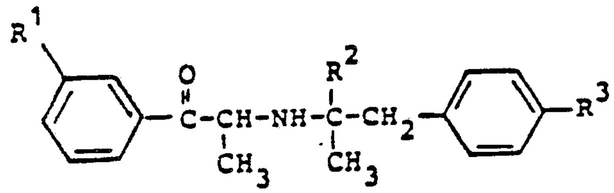

(I)

characterized in that halogenoketones of the general formula (II)

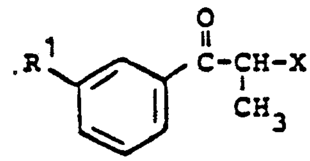

(II)

in which

$R^1$ has the abovementioned meaning and

X denotes halogen, preferably bromine,

are reacted with amines of the general formula (III)

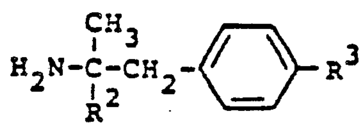

(III)

in which $R^2$ and $R^3$ have the abovementioned meaning, in the presence of inert solvents at temperatures between 0° and 150 °C, optionally in the presence of a proton acceptor, and, if appropriate, the compounds of the formula (I) thus obtained are then converted into the acid addition salts in a known manner.

6. Process according to Claim 5, characterized in that esthers, hydrocarbons, halogenohydrocarbons, ketones, nitriles, carboxamides or commercially available technical grade mixtures of these solvents are used as the inert solvent.

7. Process according to Claim 5, characterized in that at least 1 mole of the amine of the formula (III) and at least 1 mole of an acid-binding agent are employed per mole of the compound (II).

8. Medicaments containing at least one phenylethylaminopropiophenone derivative of the formula (I)

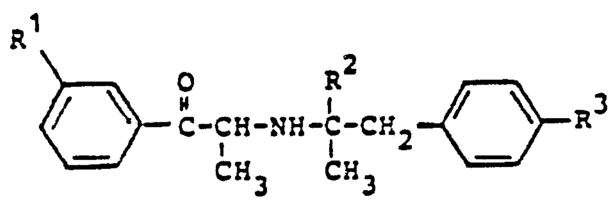

(I)

9. Use of phenylethylaminopropiophenone derivatives of the formula (I)

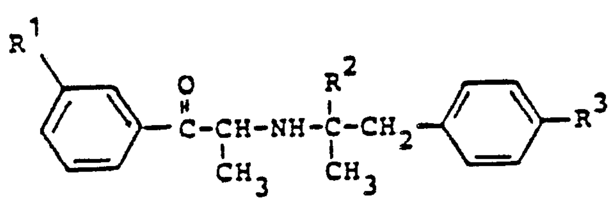

(I)

for the preparation of medicaments.

10. Phenylethylaminopropiophenone derivatives of the formula (I)

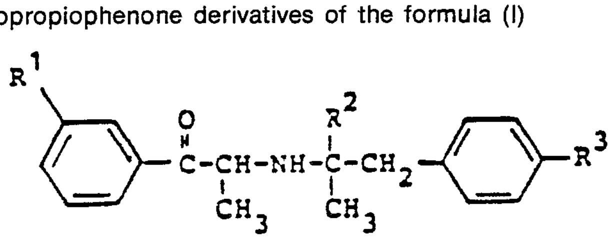

for use in combating diseases and disorders of the central nervous system.

## Revendications

1. Dérivés de la phényléthylaminopropiophénone de formule générale I :

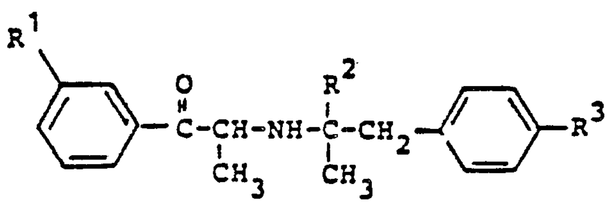

(I)

dans laquelle :

$R^1$ représente l'hydrogène, un groupe alkyle inférieur éventuellement substitué par le fluor, ou un halogène,

$R^2$ représente l'hydrogène ou un groupe alkyle inférieur, et

$R^3$ représente l'hydrogène, un groupe alcoxy ou un halogène,

et leurs sels acceptables pour l'usage pharmaceutique formés avec des acides minéraux ou organiques.

2. Dérivés de la phényléthylaminopropiophénone selon la revendication 1, dans lesquels :

$R^1$ représente l'hydrogène, un groupe alkyle inférieur en $C_1$-$C_2$, éventuellement substitué par le fluor, ou le fluor ou le chlore,

$R^2$ représente l'hydrogène, ou un groupe alkyle inférieur en $C_1$-$C_2$, et

$R^3$ représente l'hydrogène, un groupe alcoxy en $C_1$-$C_2$ ou le chlore ou le fluor.

3. Dérivés de la phényléthylaminopropiophénone selon la revendication 1, dans lesquels :

$R^1$ représente l'hydrogène, le fluor, le chlore ou un groupe trifluorométhyle,

$R^2$ représente l'hydrogène ou un groupe méthyle, et

$R^3$ représente l'hydrogène, un groupe méthoxy ou le chlore.

4. Dérivés de la phényléthylaminopropiophénone selon la revendication 1, du groupe consistant en :

le chlorhydrate de la 1-(3-chlorophényl)-2-(1-méthyl-2-phényléthylamino)-propane-1-one,

le chlorhydrate de la 1-phényl-2-(1,1-diméthyl-2-phényléthylamino)-propane-1-one,

le chlorhydrate de la 1-(3-trifluorométhylphényl)-2-(1,1-diméthyl-2-phényléthylamino)-propane-1-one,

le chlorhydrate de la 1-(3-fluorophényl)-2-(1,1-diméthyl-2-phényléthylamino)-propane-1-one,

le chlorhydrate de la 1-(3-chlorophényl)-2-(1,1-diméthyl-2-phényléthylamino)-propane-1-one,

le chlorhydrate de la 1-(3-chlorophényl)-2-(1,1-diméthyl-2-(4-chlorophényl)-éthylamino)-propane-1-one, et

le chlorhydrate de la 1-(3-chlorophényl)-2-(1,1-diméthyl-2-(4-méthoxyphényl)-éthylamino)-propane-1-one.

5. Procédé de préparation des dérivés de la phényléthylaminopropiophénone de formule générale I :

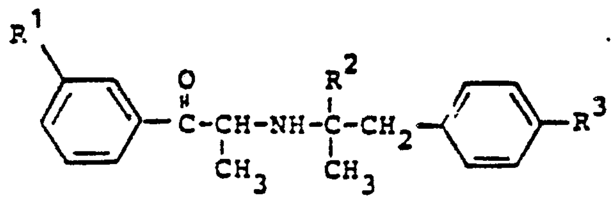

(I)

caractérisé en ce que l'on fait réagir les halogénocétones de formule générale II

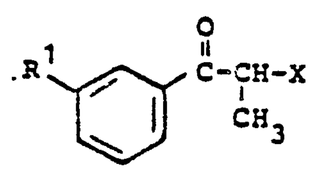

(II)

dans laquelle :

$R^1$ a les significations indiquées ci-dessus, et

X représente un halogène, de préférence le brome,

avec des amines de formule générale III :

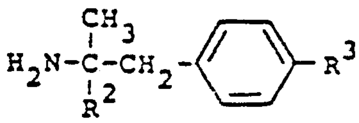

(III)

dans laquelle $R^2$ et $R^3$ ont les significations indiquées ci-dessus, en présence de solvants intertes, à des températures de 0 à 150 °C, éventuellement en présence d'un accepteur de protons, et on convertit, le cas échéant, les composés ainsi obtenus, répondant à la formule I, de manière connue en soi, en les sels formés par addition avec des acides.

6. Procédé selon la revendication 5, caractérisé en ce que l'on utilise, en tant que solvants inertes, des éthers, des hydrocarbures, des hydrocarbures halogénés, des cétones, des nitriles, des carboxamides ou des mélanges techniques de ces solvants tels qu'ils existent dans le commerce.

7. Procédé selon la revendication 5, caractérisé en ce que l'on met en œuvre au moins 1 mole de l'amine de formule III et au moins 1 mole d'un agent capteur d'acides par mole du composé II.

8. Médicament contenant au moins un dérivé de la phényléthylaminopropiophénone de formule I :

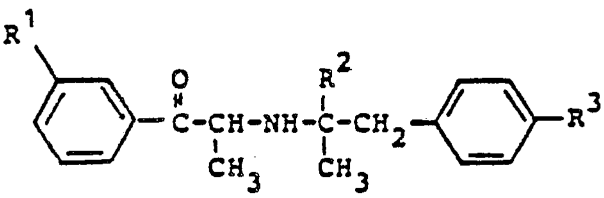

(I)

9. Utilisation des dérivés de la phényléthylaminopropiophénone de formule I :

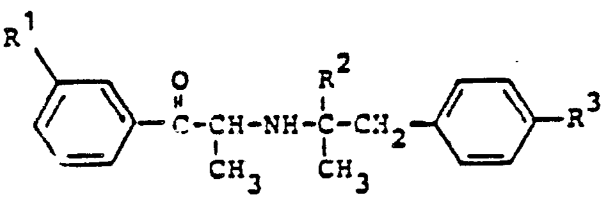

(I)

pour la préparation de médicaments.

10. Dérivés de la phényléthylaminopropiophénone de formule I :

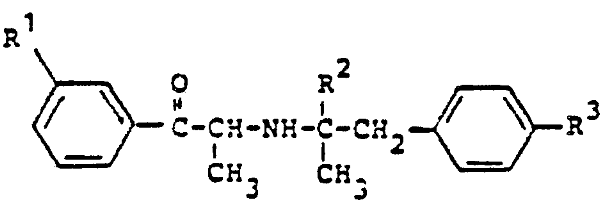

(I)

pour l'utilisation dans le traitement de maladies et des troubles du système nerveux central.